# EUROPEAN PATENT SPECIFICATION

(11) **EP 3 777 924 B1**
(45) Date of publication and mention of the grant of the patent: **03.09.2025**
(21) Application number: 19777032.4
(22) Date of filing: 27.03.2019
(51) Int. Cl.: A61M 5/145, A61M 5/142, A61M 5/28, A61M 5/31

(54) **PRE-FILLED SYRINGE, LIQUID MEDICINE ADMINISTRATION SYSTEM, AND SYRINGE PUMP**
VORGEFÜLLTE SPRITZE, SYSTEM ZUR VERABREICHUNG VON FLÜSSIGER MEDIZIN UND SPRITZENPUMPE
SERINGUE PRÉ-REMPLIE, SYSTÈME D'ADMINISTRATION DE MÉDICAMENT LIQUIDE ET POMPE À SERINGUE

(30) Priority: 29.03.2018 JP 2018066113
(43) Date of publication of application: 17.02.2021
(73) Proprietor: TERUMO Kabushiki Kaisha, Tokyo 151-0072 (JP)
(72) Inventor: OKUDA, Yuji, Fujinomiya-shi, Shizuoka 418-0004 (JP); SEKIGUCHI, Shota, Ashigarakami-gun, Kanagawa 259-0151 (JP)
(74) Representative: TBK
(86) International application number: PCT/JP2019/013334
(87) International publication number: WO 2019/189449

(56) References cited:
- EP-A1- 1 433 456
- EP-A1- 1 825 877
- EP-A1- 2 337 595
- WO-A1-2006/006643
- WO-A1-2007/032341
- WO-A1-2007/116863
- WO-A1-2017/038483
- JP-A- 2004 092 671
- JP-A- 2008 535 569
- JP-A- 2017 531 459

## Description

### Technical Field

The present disclosure relates to a syringe pump to which a prefilled syringe is mounted.

### Background Art

When a medicine such as an intravenous anesthetic is delivered into the patient's body in a medical setting such as an operating room or an intensive care unit, it is necessary to deliver the medicine over a long time while adjusting a flow rate (hereinafter also simply referred to as a "liquid delivery amount") of the medicine to be delivered according to a technique to be applied, patient's condition, and the like. As a device for accurately delivering a medicine over a long period of time with a set liquid delivery amount, a liquid medicine administration device, such as a syringe pump, capable of delivering a liquid medicine containing a medicine using a prefilled syringe that contains the liquid medicine in a barrel has been known, for example.

Document JP 2015-217176 A discloses a liquid medicine injection device as a liquid medicine administration device of this type. JP 2015-217176 A also discloses a syringe which is to be mounted on the liquid medicine injection device and which has a radio frequency identification (RFID) tag attached thereto, the RFID tag having various kinds of data recorded thereon. Further, the liquid medicine injection device disclosed in JP 2015-217176 A has an RFID module including a reader that acquires various kinds of recorded data from the RFID tag of the syringe. Document EP 1 433 456 A1 discloses a drug container and an injector comprising the same. When using the drug container having an identification tag fixed or detachably provided at a predetermined position of the container, the tag having drug data on a kind and a concentration of a drug and both or one of upper and lower limits of a flow rate on a continuous infusion and the upper and lower limits, time and flow rate on a one-shot administration recorded thereon, it is possible to ensure safety by prompting for a stop of injection by a warning when a setting beyond the upper and lower limits is performed and rewrite a liquid infusing time and a flow rate and so on required to be set according to symptoms of a patient.

EP 1 825 877 A1 pertains to a chemical solution injection system, wherein a RFID chip is mounted on a chemical liquid syringe in the state of being wrapped around the outer surface of the cylinder member thereof. An amplification antenna wrapped one turn around the outer surface of the cylinder member is disposed at the front of the RFID chip parallel with each other. Since the communication performance of the RFID chip is increased by the amplifying antenna, the RFID chip of the chemical solution syringe is allowed to satisfactorily communicate with the RFID reader of injection head.

WO 2007/116 863 A1 discloses a medicinal-liquid injection system. Here, An RFID tag is attached to a cylinder body of a medicinal-liquid syringe. A medicinal-liquid injection device has a body holding member made of metal and holding the cylinder body on its upper surface. An RFID reader is placed on the lower surface of the body holding member. A holding recess for holding the cylinder body is provided in the body holding member, and a through-hole is formed in the holding recess. An antenna sheet is inserted in the through-hole. One end of the antenna sheet is placed on the inner surface of the holding recess and the other end is faced to the RFID reader.

In EP 2 337 595 A1 A power injector syringe clamp assembly is disclosed. This clamp assembly includes a first clamp member and a second clamp member, where at least one of these clamp members is movable to provide open and closed configurations for the clamp assembly. The clamp assembly also includes at least one RFID reader antenna for communicating with at least one RFID tag on a power injector syringe, at least when positioned within the clamp assembly.

### Summary of Invention

### Technical Problem

When the liquid medicine injection device disclosed in JP 2015-217176 A is used, the syringe is mounted on a base part of the liquid medicine injection device in a medial setting. When the syringe is mounted, it is necessary to bring the RFID tag close to the reader of the liquid medicine injection device so that the reader of the liquid medicine injection device can acquire the data of the RFID tag of the syringe. Therefore, it is necessary to mount the cylindrical syringe on the base part of the liquid medicine injection device while the RFID tag of the syringe is aligned with the reader of the liquid medicine injection device. This work is complicated for a medical staff. Further, when the positional alignment described above is performed by rotating the syringe about the axis thereof with the syringe being mounted on the base part, the RFID tag may be damaged due to a sliding movement between the RFID tag fixed on the outer circumferential surface of the syringe and the base part of the liquid medicine injection device.

Further, when the RFID tag is attached to the outer circumferential surface of the syringe, the attached RFID tag may be deformed, because the outer circumferential surface of the syringe is curved. Due to the deformation, the RFID tag may be detached.

It is the object of the present invention to provide a syringe pump adapted to a prefilled syringe including an RFID tag from which data is easily acquired by a syringe pump and which is less likely to be detached, and a liquid medicine administration system.

The object of the invention is achieved by a syringe pump according to claim 1. Advantageous embodiments are carried out according to the dependent claim.

A prefilled syringe according includes: a liquid medicine; a barrel including a body section that is cylindrical and that contains the liquid medicine, and a nozzle section that is provided on a distal end side of the body section and that discharges the liquid medicine; a cap that seals a distal end opening provided on a distal end section of the nozzle section; a gasket that slides on an inner circumferential surface of the body section; a plunger mountable to the gasket; and an RFID tag that has a fixed location with respect to an outer circumferential surface of the body section and has an antenna for communication and a memory, the antenna being structured from an antenna wire that is wound in a rectangle, wherein an outer diameter of the body section of the barrel is 14 mm to 33 mm, a maximum circumferential length of the antenna of the RFID tag along a circumferential direction of the body section of the barrel is 9 mm to 25 mm, and a circumferential length of the outer circumferential surface of the body section of the barrel is 2.0 to 7.0 times the maximum circumferential length of the antenna of the RFID tag.

A maximum axial length of the RFID tag along an axial direction of the body section of the barrel can be 8 mm to 25 mm.

The barrel can have a flange that protrudes from a proximal end section of the body section, the body section has, near the flange, a clamp contact portion where a clamp section of a syringe pump that drives the plunger contacts, and the RFID tag is disposed distal to the clamp contact portion in the axial direction of the body section.

A distance between a proximal end of the RFID tag and a distal end of the flange of the barrel can be 15 mm to 40 mm in the axial direction of the body section.

The RFID tag can be located proximal to the distal end of the gasket in the axial direction of the body section.

The prefilled syringe further can include an information label attached to the body section of the barrel, wherein the RFID tag is attached on an inner surface or an outer surface of the information label.

The prefilled syringe further can include an information label attached to the body section of the barrel so as to cover an outer surface of the RFID tag.

The information label can have a scale indicating an amount of the liquid medicine in the body section, the scale being provided along an axial direction of the body section of the barrel, and at least a part of the RFID tag is located at a position overlapping with at least a part of the scale in the axial direction of the body section of the barrel and displaced in the circumferential direction of the body section of the barrel.

The scale can include a plurality of scales, and the RFID tag is disposed between at least two of the scales.

The at least two scales are symmetrical with respect to a central axis of the body section of the barrel.

A liquid medicine administration system according to the present invention is a syringe pump to which a prefilled syringe as described above is mounted, the prefilled syringe including a liquid medicine, a barrel including a body section that is cylindrical and that contains the liquid medicine, and a nozzle section that is provided on a distal end side of the body section and that discharges the liquid medicine, a cap that seals a distal end opening provided on a distal end section of the nozzle section, a gasket that slides on an inner circumferential surface of the body section, a plunger mountable to the gasket, and an RFID tag that has a fixed location with respect to an outer circumferential surface of the body section and has an antenna for communication and a memory, the antenna being structured from an antenna wire that is wound in a rectangle. An outer diameter of the body section of the barrel is 14 mm to 33 mm, a maximum circumferential length of the antenna of the RFID tag along a circumferential direction of the body section of the barrel is 9 mm to 25 mm, and a circumferential length of the outer circumferential surface of the body section of the barrel is 2.0 to 7.0 times the maximum circumferential length of the antenna of the RFID tag. The syringe pump includes: a syringe plunger driving section that drives the plunger of the prefilled syringe in a distal direction toward the distal end of the body section; a main body provided with a reader and a control unit that controls the syringe plunger driving section, the reader reading a data set stored in the memory of the RFID tag of the prefilled syringe; a supporting section that supports the outer circumferential surface of the body section of the prefilled syringe in a direction perpendicular to an axis of the body section; and a clamp section that faces the supporting section and clamps the body section of the prefilled syringe with the supporting section, wherein the main body of the syringe pump has a first region facing the clamp section and a second region that is adjacent to the first region and is located on a side opposite to the syringe plunger driving section in an axial direction of the body section, the reader includes a reader antenna that communicates with the antenna of the RFID tag and that is structured from a reader antenna wire, and at least a part of the reader antenna is located in the second region.

A centerline of the reader antenna perpendicular to the axial direction of the body section of the barrel can be located in the second region in a front view of the supporting section.

### Advantageous Effects of Invention

The present disclosure can provide syringe pump adapted to a prefilled syringe including an RFID tag from which data is easily acquired by a syringe pump and which is less likely to be detached.

### Brief Description of Drawings

Fig. 1 is a perspective view showing a liquid medicine administration system including a prefilled syringe and a syringe pump as one embodiment.
Fig. 2 is a perspective view showing the prefilled syringe shown in Fig. 1.
Fig. 3 is a view showing an information label to be attached to the prefilled syringe shown in Fig. 2.
Fig. 4 is a block diagram showing an RFID tag attached to the prefilled syringe and a reader of the syringe pump.
Fig. 5 is a diagram showing an example of a method for obtaining a prefilled syringe having an information label attached thereto.
Fig. 6 is an enlarged front view in which a part of the front view of a main body of the syringe pump shown in Fig. 1 is enlarged.
Fig. 7 is a sectional view of the prefilled syringe and the syringe pump shown in Fig. 1.
Fig. 8 is a perspective view showing a prefilled syringe according to another embodiment.

### Description of Embodiments

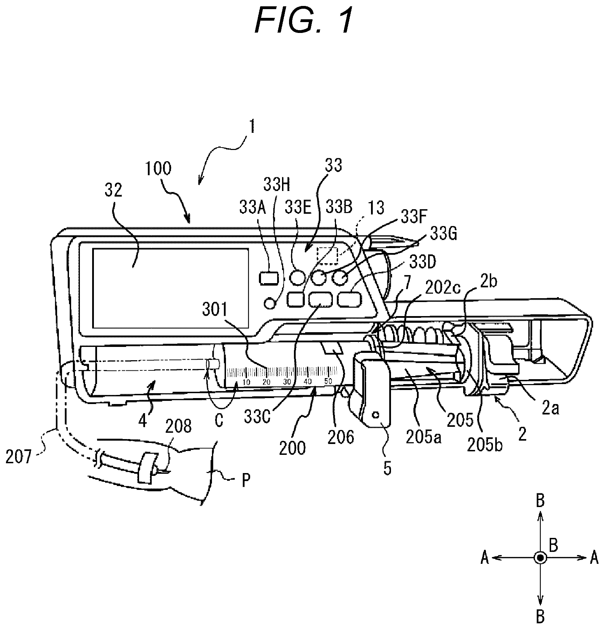

Hereinafter, embodiments of a prefilled syringe, a liquid medicine administration system, and a syringe pump according to the present disclosure will be described with reference to Figs. 1 to 8. In the drawings, same members and parts are denoted by the same reference numerals.

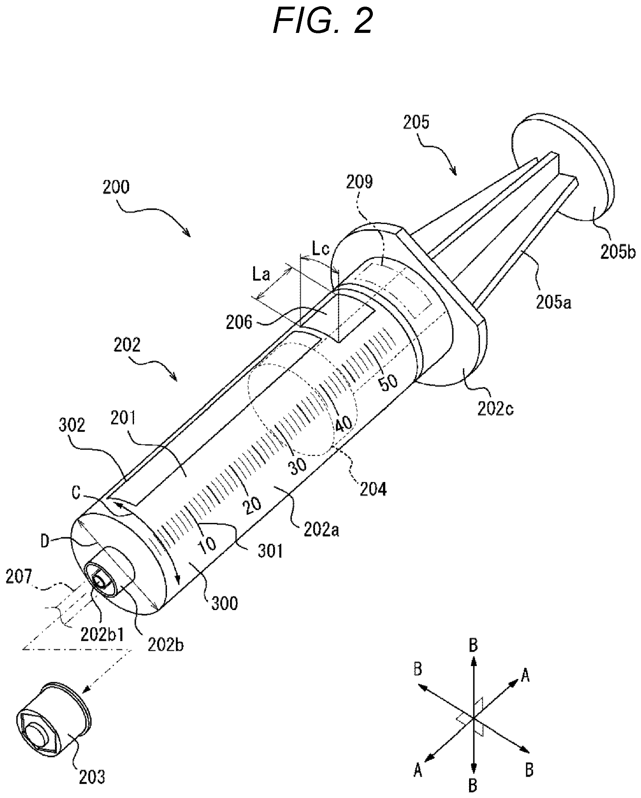

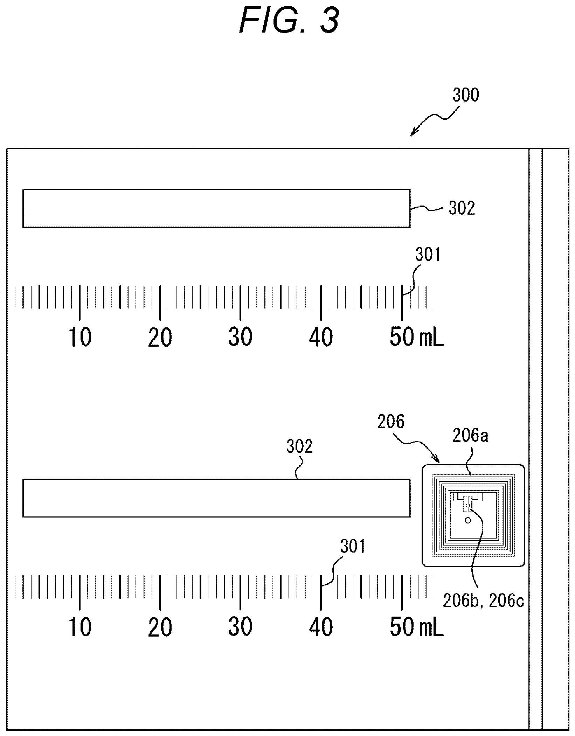

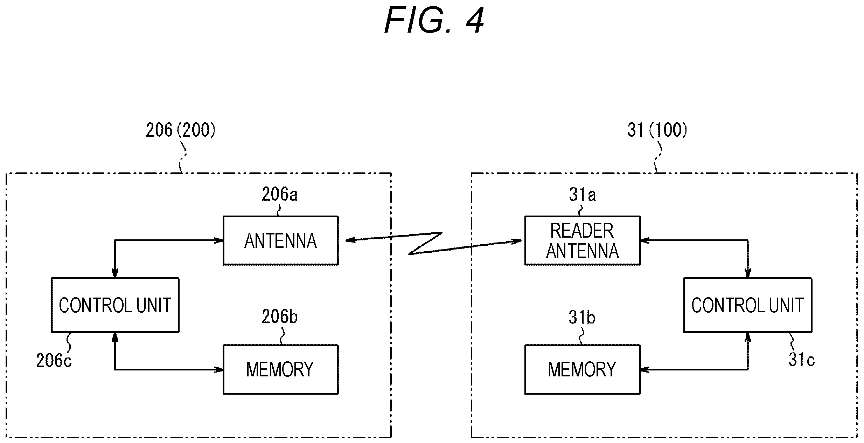

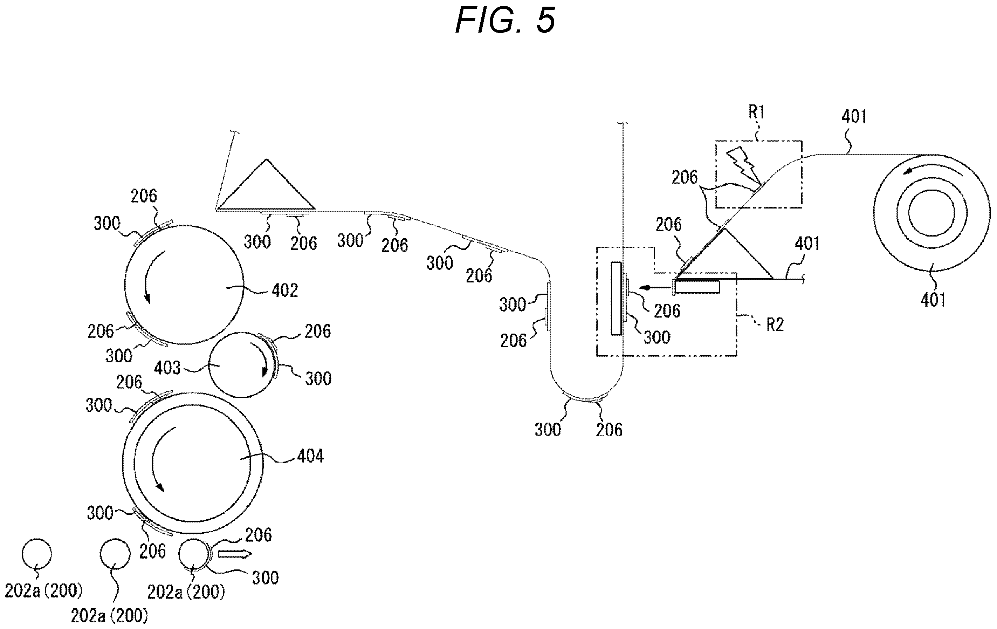

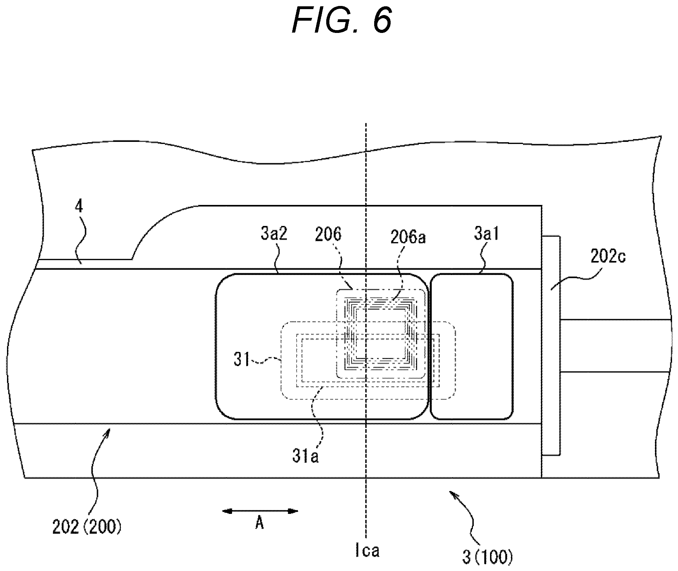

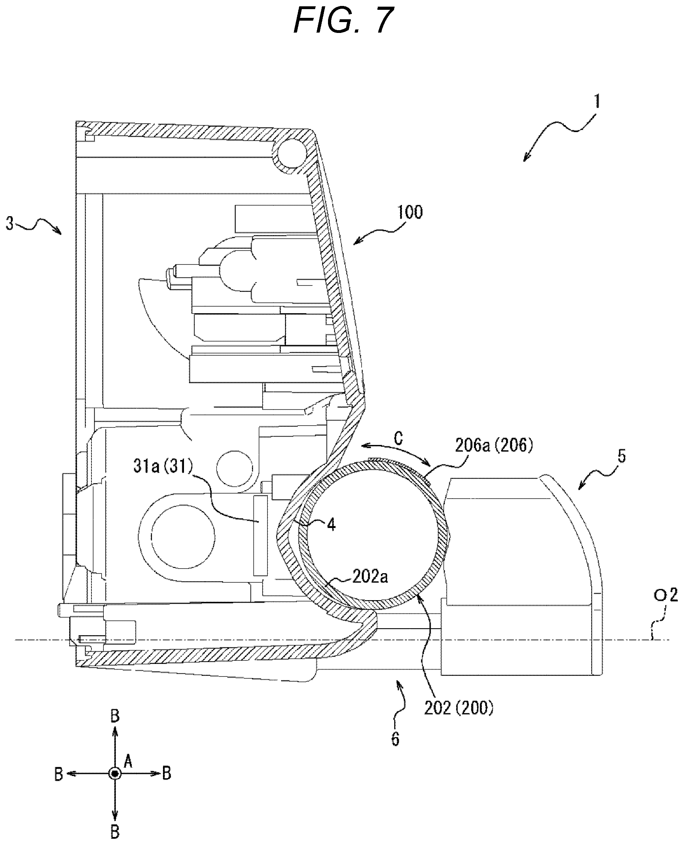

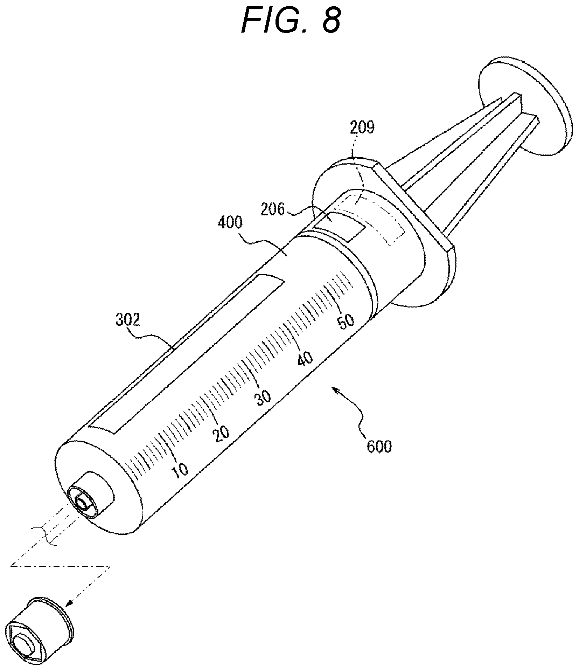

Fig. 1 is a perspective view showing a liquid medicine administration system 1 including a prefilled syringe 200 and a syringe pump 100 according to one embodiment. Fig. 2 is a perspective view showing the prefilled syringe 200 shown in Fig. 1. Fig. 3 is a view showing an information label 300 to be attached to the prefilled syringe 200 shown in Fig. 2. Fig. 4 is a block diagram showing an RFID tag 206 attached to the prefilled syringe 200 and a reader 31 of the syringe pump 100. Fig. 5 is a diagram showing an example of a method for obtaining the prefilled syringe 200 having the information label 300 attached thereto. Fig. 6 is an enlarged front view in which a part of the front view of a main body 3 of the syringe pump 100 shown in Fig. 1 is enlarged. Fig. 7 is a sectional view of the prefilled syringe 200 and the syringe pump 100 shown in Fig. 1. More specifically, Fig. 7 is a sectional view showing a cross section perpendicular to the axial direction of the prefilled syringe 200 in a state where the prefilled syringe 200 is mounted on the syringe pump 100. Fig. 8 is a perspective view showing a prefilled syringe 600 according to another embodiment.

As shown in Fig. 1, the liquid medicine administration system 1 according to the present embodiment includes the syringe pump 100 and the prefilled syringe 200 that can be mounted to the syringe pump 100. Each component will be described below.

### [Prefilled syringe 200]

First, the prefilled syringe 200 will be described in detail. As shown in Fig. 2, the prefilled syringe 200 includes a liquid medicine 201, a barrel 202, a cap 203, a gasket 204, a plunger 205, and an RFID tag 206.

The liquid medicine 201 is, for example, an anticancer agent, an anesthetic, a chemotherapeutic agent, a blood transfusion, a nutrient, or the like. The liquid medicine 201 is contained in the barrel 202. The liquid medicine 201 is microinjected into the patient's body as described later.

The barrel 202 includes a cylindrical body section 202a, a nozzle section 202b, and a flange 202c.

The body section 202a contains the liquid medicine 201. An outer diameter D of the body section 202a is 14 mm to 33 mm. Further, the circumferential length of the outer circumferential surface of the body section 202a is 2.0 to 7.0 times the maximum circumferential length Lc of a later-described antenna of the RFID tag 206.

The nozzle section 202b is provided on the distal end side of the body section 202a. A distal end opening 202b1 is provided at a distal end section of the nozzle section 202b. The liquid medicine 201 is expelled from the distal end opening 202b1. A tube 207 (indicated by a chain double-dashed line in Figs. 1 and 2) can be connected to the nozzle section 202b.

The flange 202c protrudes outward from the proximal end section of the body section 202a in the radial direction B of the body section 202a.

The cap 203 can seal the distal end opening 202b1 provided at the distal end section of the nozzle section 202b. The cap 203 can be attached to the nozzle section 202b, and Fig. 2 shows a state in which the cap 203 is removed from the nozzle section 202b and the distal end opening 202b1 is opened.

The gasket 204 slides on the inner circumferential surface of the body section 202a. The gasket 204 in the present embodiment is a tubular body having a closed distal end and an open proximal end. The gasket 204 constitutes a liquid medicine containing section in which the liquid medicine 201 is contained in the barrel 202. In the present embodiment, an amount of the liquid medicine to be contained is 5 ml to 50 ml. Note that, in general, an amount (ml) of liquid medicine to be contained is associated with the outer diameter D (mm) of the prefilled syringe 200, and is specified in, for example, ISO 11040-6 (Prefilled syringes-Part 6: Plastic barrels for injectables). In the present embodiment, when a prefilled syringe that can contain 5 ml of liquid medicine is used, the outer diameter D is 14 mm. In addition, when the amount of the liquid medicine that can be contained is 10 ml, the outer diameter D is 17 mm. When the amount of the liquid medicine that can be contained is 20 ml, the outer diameter D is 22 mm. Further, when the amount of the liquid medicine that can be contained is 50 ml, the outer diameter D is 33 mm.

The plunger 205 can be mounted to the gasket 204. The plunger 205 includes a plunger body 205a and a flange 205b. In the present embodiment, the distal end section of the plunger body 205a can be fixed to the gasket 204 while being inserted from the proximal end side of the gasket 204. The plunger body 205a and the gasket 204 can be fixed to each other by, for example, thread connection. The plunger body 205a is inserted into the body section 202a and is movable in the axial direction A of the body section 202a within the body section 202a. The flange 205b protrudes outward from the plunger body 205a in the radial direction B on the proximal end section of the plunger 205.

In the present embodiment, the RFID tag 206 is attached to the outer surface of the information label 300 described later. That is, the RFID tag 206 in the present embodiment is attached to the outer circumferential surface of the body section 202a via the information label 300. In another embodiment, the information label 300 may be attached to the body section 202a of the barrel 202 so as to cover the outer surface of the RFID tag 206.

The RFID tag 206 is not attached to the body section 202a over the entire circumference in the circumferential direction C, but is attached only to a part of the body section 202a in the circumferential direction C.

In the present embodiment, the maximum axial length La of the RFID tag 206 along the axial direction A of the body section 202a of the barrel 202 is 8 mm to 25 mm. In the present embodiment, the proximal end of the RFID tag 206 is located distant from the distal end of the flange 202c of the barrel 202 by 15 mm to 40 mm (for example, 24.8 mm) in a distal direction toward the distal end of the body section 202a in the axial direction A. In the present embodiment, the RFID tag 206 is located proximal to the distal end of the gasket 204 in the axial direction A of the body section 202a. Specifically, the RFID tag 206 in the present embodiment is located proximal to the distal end of the gasket 204 of the unused prefilled syringe 200 in the axial direction A of the body section 202a. In other words, the RFID tag 206 in the present embodiment is not located further distal to the distal end of the gasket 204 in the axial direction A of the body section 202a. As shown in Fig. 1, in the present embodiment, a portion (hereinafter referred to as a clamp contact portion 209) clamped by a clamp section 5 of the syringe pump 100 in the outer circumferential surface of the prefilled syringe 200 is located proximal to the portion where the RFID tag 206 is attached.

For example, the RFID tag 206 can be formed into a rectangle having a circumferential length Lc of 18 mm and an axial length La of 18 mm in a state where the position is fixed with respect to the outer circumferential surface of the body section 202a. Alternatively, the RFID tag 206 may be formed into a rectangle having a circumferential length Lc of 25 mm and an axial length La of 25 mm in the same state. The RFID tag 206 may also be formed into a rectangle having a circumferential length Lc of 12 mm and an axial length La of 9 mm in the same state.

As shown in Figs. 3 and 4, the RFID tag 206 has an antenna 206a for communication, a memory 206b, and a control unit 206c.

As shown in Fig. 3, the antenna 206a of the RFID tag 206 is structured from an antenna wire wound in a rectangle. Although the area on the body section 202a where the RFID tag 206 can be installed is limited as shown in Fig. 2, a loop area formed by the antenna wire is easily ensured by winding the antenna wire in a rectangle, as compared with a configuration in which the antenna wire is wound in a circle. Thus, even if the RFID tag 206 of the prefilled syringe 200 and the reader 31 of the syringe pump 100 are not aligned with high precision when the prefilled syringe 200 is mounted on a supporting section 4 of the syringe pump 100, the RFID tag 206 can communicate with the reader 31 as shown in Figs. 1 and 7.

The antenna 206a of the RFID tag 206 performs communication by wireless communication having a short working distance such as near field communication (NFC).

While the RFID tag 206 has a fixed location with respect to the outer circumferential surface of the body section 202a of the barrel 202, the maximum circumferential length Lc of the antenna 206a of the RFID tag 206 along the circumferential direction C of the body section 202a of the barrel 202 is 9 mm to 25 mm. For example, the outer perimeter of the antenna 206a can be formed into a rectangle having a circumferential length of 15 mm and an axial length of 15 mm in the abovementioned state. Alternatively, the outer perimeter of the antenna 206a may be formed into a rectangle having a circumferential length of 25 mm and an axial length of 25 mm in the abovementioned state.

The control unit 206c of the RFID tag 206 can read data from the memory 206b and cause the antenna 206a to transmit the data. In the wireless communication between the antenna 206a of the RFID tag 206 and a reader antenna 31a of the reader 31 of the syringe pump 100, a communicable distance is short (for example, within 35 mm). Therefore, when the RFID tag 206 of the prefilled syringe 200 is distant from the reader antenna 31a of the reader 31 by a predetermined distance or more, the reader antenna 31a of the reader 31 cannot communicate with the RFID tag 206 of the prefilled syringe 200. The memory 206b and the control unit 206c of the RFID tag 206 can be constituted by, for example, an integrated circuit (IC chip) including a nonvolatile memory.

As shown in Fig. 4, the antenna 206a of the RFID tag 206 receives an electromagnetic wave transmitted from the reader antenna 31a of the reader 31 of the syringe pump 100. The operating power of the RFID tag 206 can be obtained from this electromagnetic wave. The control unit 206c reads the data in the memory 206b of the RFID tag 206, and sends (transmits) the data to the reader antenna 31a of the reader 31 through the electromagnetic wave using the antenna 206a. The reader antenna 31a of the reader 31 receives the electromagnetic wave from the antenna 206a of the RFID tag 206. Then, the control unit 31c of the syringe pump 100 acquires the data stored in the memory 206b of the RFID tag 206 by extracting the data from the received electromagnetic wave, and stores the data in the storage unit 31b of the syringe pump 100.

The memory 206b of the RFID tag 206 stores, for example, various kinds of data regarding the prefilled syringe 200, such as the name of the liquid medicine 201, identification data for each prefilled syringe, dimensional data of the barrel 202, and dimensional data of the stroke of the plunger 205.

As shown in Fig. 2, the information label 300 is attached to the outer circumferential surface of the body section 202a of the barrel 202. Fig. 3 shows the information label 300 which is not yet attached to the outer circumferential surface of the body section 202a.

The information label 300 is provided with two scales 301 which extend along the axial direction A of the body section 202a when the information label 300 is attached to the outer circumferential surface of the body section 202a (see Fig. 2). More specifically, the notches of the two scales 301 in the present embodiment are arranged along the axial direction A of the body section 202a when the information label 300 is attached to the outer circumferential surface of the body section 202a. The scales 301 indicate the amount of the liquid medicine 201 in the body section 202a when the information label 300 is attached to the outer circumferential surface of the body section 202a. In the present embodiment, when the information label 300 is attached to the outer circumferential surface of the body section 202a of the barrel 202 as shown in Fig. 2, the two scales 301 are symmetrical with respect to the central axis of the body section 202a.

As shown in Fig. 3, the RFID tag 206 is attached between the two scales 301 of the information label 300. In the present embodiment, the RFID tag 206 is attached to the outer surface of the information label 300. In another embodiment, the RFID tag 206 can be attached to the inner surface of the information label 300.

In the present embodiment, a part of the RFID tag 206 is located at a position overlapping with a part of the two scales 301 in the longitudinal direction of the information label 300 and displaced in the transverse direction of the information label 300. That is, when the information label 300 is attached to the outer circumferential surface of the body section 202a as shown in Fig. 2, a part of the RFID tag 206 is located at a position overlapping with a part of the scales 301 in the axial direction A of the body section 202a of the barrel 202 and displaced in the circumferential direction C of the body section 202a of the barrel 202.

The information label 300 can be provided with an information area 302 in which the name of the medicine or an amount of the liquid medicine is written, in addition to the scales 301.

An example of a method for obtaining the prefilled syringe 200 to which the information label 300 is attached will be described with reference to Fig. 5. First, a roll-shaped mount 401 is pulled out. RFID tags 206 are mounted on the mount 401 at regular intervals. Next, various kinds of information are written in the RFID tag 206 as indicated by an area R1. Thereafter, as indicated by an area R2, the RFID tag 206 is removed from the mount 401, and the removed RFID tag 206 is attached to the outer surface of the information label 300. Then, the orientation of the information label 300 is properly set on a drum 402. Then, it is checked whether the information written in the RFID tag 206 is correct on a drum 403. Finally, the information label 300 is attached to the outer circumferential surface of the body section 202a of the prefilled syringe 200 using a drum 404.

### [Syringe pump 100]

Next, the syringe pump 100 will be described with reference to Figs. 1, 6, and 7.

The syringe pump 100 is used in, for example, an intensive care unit. Further, the syringe pump 100 can be used when a liquid medicine such as an anticancer agent, an anesthetic, a chemotherapeutic agent, a blood transfusion, or a nutrient is microinjected to a patient P over a relatively long time with high accuracy.

Further, the syringe pump 100 according to the present embodiment can be mounted to and removed from a stand or the like, and can be used while being mounted on the stand or the like. The syringe pump 100 is fixed such that the axial direction A of the body section 202a of the prefilled syringe 200 mounted on the syringe pump 100 coincides with the horizontal direction.

As shown in Fig. 1, the syringe pump 100 includes a syringe plunger driving section 2, a main body 3, a supporting section 4, and a clamp section 5.

The syringe plunger driving section 2 drives the plunger 205 of the prefilled syringe 200 in the distal direction toward the distal end of the body section 202a.

The syringe plunger driving section 2 in the present embodiment includes a pressing part 2a and a flange fixing part 2b.

The pressing part 2a is located proximal to the flange 205b of the plunger 205 of the mounted prefilled syringe 200 in the axial direction A. Then, when the pressing part 2a is moved toward the distal side in the axial direction A, the surface of the flange 205b on the proximal side in the axial direction A can be pressed toward the distal side in the axial direction A. Accordingly, the plunger 205 can be relatively moved to the distal side in the axial direction A with respect to the barrel 202 of the mounted prefilled syringe 200.

The flange fixing part 2b fixes the flange 205b of the plunger 205 to the pressing part 2a. Specifically, the flange fixing part 2b in the present embodiment is located on the distal side of the pressing part 2a in the axial direction A and is attached to the pressing part 2a. While the prefilled syringe 200 is mounted, the flange 205b of the plunger 205 is located between the pressing part 2a and the flange fixing part 2b. Thus, the plunger 205 is movable in the axial direction A with the movement of the syringe plunger driving section 2 in the axial direction A.

Fig. 6 is an enlarged front view in which a part of the front view of the main body 3 of the syringe pump 100 is enlarged. More specifically, Fig. 6 is an enlarged view of a position of the later-described supporting section 4 that receives the prefilled syringe 200 in the main body 3. Note that, for convenience of description, Fig. 6 shows the barrel 202 of the prefilled syringe 200 which is appropriately received by the supporting section 4 with the flange 202c being engaged with a flange receiving groove 7 (see Fig. 1) of the main body 3. The main body 3 is provided with the reader 31 including the reader antenna 31a that receives a data set stored in the memory of the RFID tag 206 of the prefilled syringe 200, and a control unit 13 (see Fig. 1) that controls the syringe plunger driving section 2. Specifically, the reader 31 and the control unit 13 in the present embodiment are arranged inside the main body 3. Note that the control unit 13 of the syringe pump 100 and the control unit 31c described above can be provided as separate components. Further, the control unit 31c and the control unit 13 may be integrated as the same component. In the present embodiment, the reader antenna 31a of the reader 31 is structured from a reader antenna wire. As shown in Fig. 6, the outer perimeter of the reader antenna 31a structured from the reader antenna wire is rectangular. In the present embodiment, a part of the reader antenna 31a of the reader 31 is displaced from a later-described first region 3a1 that faces the later-described clamp section 5 (see Fig. 1) of the syringe pump 100 in the axial direction A in a front view (same as the front view of the main body 3 in Fig. 6) of the supporting section 4 of the syringe pump 100.

In the present embodiment, the main body 3 has the first region 3a1 that faces the clamp section 5, and a second region 3a2 that is adjacent to the first region 3a1 on the side opposite to the syringe plunger driving section 2 in the axial direction A of the body section 202a. Then, at least a part of the reader antenna 31a of the reader 31 is located in the second region 3a2. Further, in the present embodiment, in the front view of the supporting section 4 (the same as the front view of the main body 3 in the present embodiment), the centerline lca of the reader antenna 31a, which is perpendicular to the axial direction A of the body section 202a of the barrel 202, is located in the second region 3a2. Furthermore, in the present embodiment, the centerline lca of the reader antenna 31a overlaps with the antenna 206a of the RFID tag 206 in the front view of the supporting section 4. Note that, since the outer perimeter of the reader antenna 31a is rectangular in the present embodiment, the centerline lca of the reader antenna 31a is a line passing through the centers of two sides along the axial direction A of the body section 202a of the barrel 202 among the sides defining the perimeter of the reader antenna 31a. In other words, the centerline lca of the reader antenna 31a is a line perpendicular to the axial direction A of the body section 202a of the prefilled syringe 200 and bisecting the loop area of the reader antenna 31a. If the perimeter of the reader antenna is not rectangular, a line that is perpendicular to the axial direction of the body section of the prefilled syringe and that bisects the loop area of the reader antenna may be used as the centerline.

The reader antenna 31a of the reader 31 can emit an electromagnetic wave in a state where the prefilled syringe 200 is received by the later-described supporting section 4. The RFID tag 206 attached to the prefilled syringe 200 transmits data in response to the electromagnetic wave. The reader antenna 31a of the reader 31 can receive the data.

Further, as shown in Fig. 1, the main body 3 in the present embodiment includes a display unit 32 and an operation panel 33.

The display unit 32 is an image display device capable of color display. The display unit 32 can be composed of, for example, a color liquid crystal display device. The display unit 32 can display not only information in Japanese but also information in a plurality of foreign languages as needed. The display unit 32 in the present embodiment is provided above the later-described supporting section 4 in the front surface of the syringe pump 100.

The display unit 32 may include an input device such as a touch sensor and may receive an input from the user.

The operation panel 33 is disposed above the supporting section 4 and on the right of the display unit 32 in the front surface of the syringe pump 100. The operation panel 33 includes a power switch 33A, an operation indicator 33H, and operation switches. Fig. 1 shows a fast delivery switch 33B, a start switch 33C, a stop switch 33D, a display changeover switch 33E, a return/mute switch 33F, and a confirmation switch 33G as an example of the operation switches.

The operation on the operation panel 33 described above is input to the control unit 13, and each section of the syringe pump 100 operates according to a command from the control unit 13.

Further, the main body 3 in the present embodiment includes inside various wires for electrically connecting the components of the syringe pump 100, various mechanisms for executing commands from the control unit, a communication unit capable of wireless or wired communication with external devices other than the RFID tag 206, a storage unit that stores various kinds of data and various programs necessary for the operation of the syringe pump 100, and the like.

As shown in Fig. 1, the supporting section 4 in the present embodiment is formed on the front surface of the main body 3. Further, the supporting section 4 in the present embodiment supports the outer circumferential surface of the body section 202a of the prefilled syringe 200 in a direction perpendicular to the central axis of the body section 202a. As shown in Fig. 7, the supporting section 4 in the present embodiment is constituted by a concave curved surface having a substantially semicircular cross section in order to receive the outer circumferential surface of the body section 202a. Further, the supporting section 4 in the present embodiment can receive a plurality of types of body sections 202a having different sizes such as outer diameters.

As shown in Fig. 1, the clamp section 5 faces the supporting section 4 formed on the main body 3 and clamps the body section 202a of the prefilled syringe 200 with the supporting section 4. In the present embodiment, the clamp contact portion 209 (see Fig. 2) which is a portion of the outer circumferential surface of the body section 202a where the clamp section 5 clamps the prefilled syringe 200 is different from the portion where the RFID tag 206 is attached, and is located proximal to the portion where the RFID tag 206 is attached.

As shown in Fig. 7, the clamp section 5 is supported by a base 6 so as to be movable in the longitudinal direction of the base 6 (direction parallel to the central axis O2). Therefore, the distance between the clamp section 5 and the supporting section 4 provided on the front surface of the main body 3 can be changed by moving the clamp section 5 in the longitudinal direction of the base 6. This makes it possible to move the clamp section 5 between a clamping position and a non-clamping position.

The clamping position of the clamp section 5 indicates a position where the body section 202a of the prefilled syringe 200 is clamped between the clamp section 5 and the supporting section 4 as shown in Fig. 7. Further, the non-clamping position of the clamp section 5 indicates a position where the clamp section 5 is moved further away from the supporting section 4 with respect to the clamp position so as not to clamp the body section 202a with the supporting section 4.

The clamp section 5 is constantly biased by a biasing member such as a spring member in a direction approaching the supporting section 4 in the longitudinal direction of the base 6. Therefore, when the clamp section 5 is moved from the clamping position to the non-clamping position, the clamp section 5 is moved against the biasing force of the biasing member.

Further, the clamp section 5 is supported by the base 6 so as to be movable not only in the longitudinal direction of the base 6 but also in the circumferential direction around the central axis O2 of the base 6. As a result, the position of the base 6 in the longitudinal direction can be fixed so that the clamp section 5 does not move in the longitudinal direction of the base 6. Specifically, the clamp section 5 is rotatable in the circumferential direction around the central axis O2 of the base 6 between a position facing the supporting section 4 and a position not facing the supporting section 4. This rotation operation is disabled when the clamp section 5 is at the clamping position, and is enabled when the clamp section 5 is at the non-clamping position. The clamp section 5 can be set to have an angle by which the clamp section 5 does not face the supporting section 4, and in this state, the prefilled syringe 200 can be easily placed on the receiving surface of the supporting section 4.

The prefilled syringe 200 operates as follows in the mounted state (see Fig. 1) mounted on the syringe pump 100. The plunger 205 is pressed toward the distal side in the axial direction A by the syringe pump 100. As a result, the gasket 204 connected to the plunger 205 slides toward the distal side in the axial direction A within the body section 202a of the barrel 202.

When the gasket 204 slides toward the distal side in the axial direction A within the body section 202a, the liquid medicine 201 in the body section 202a is compressed. The liquid medicine 201 is expelled through the nozzle section 202b of the body section 202a by the compressive force. When the syringe pump 100 is used, the tube 207 (see Fig. 1) is connected to the distal end opening 202b1 of the nozzle section 202b of the prefilled syringe 200. Further, as shown in Fig. 1, an indwelling needle 208 to be indwelled in the patient P is connected to the distal end of the tube 207. Therefore, the liquid medicine 201 in the body section 202a can be delivered into the body of the patient P through the tube 207 and the indwelling needle 208.

Fig. 8 shows a prefilled syringe 600 according to another embodiment. Hereinafter, the prefilled syringe 600 will be described as compared with the prefilled syringe 200 shown in Fig. 2. The longitudinal length ratio of an information label 400 in Fig. 8 is smaller than the longitudinal length ratio of the information label 300 in Fig. 2. The longitudinal length ratio means the ratio of the total length of the information label in the axial direction A to the total length of the barrel in the axial direction A of the body section. The RFID tag 206 in Fig. 8 has a configuration same as the configuration shown in Fig. 2. However, the distance (for example, 24.8 mm) between the flange 202c and the RFID tag 206 in the axial direction A in Fig. 2 is greater than the distance (for example, 11 mm) between the flange 202c and the RFID tag 206 in the axial direction A in Fig. 6. Note that, in the present embodiment, the clamp contact portion 209 of the syringe pump 100 is also located proximal to the portion where the RFID tag 206 is attached in Fig. 8. The other configuration of the prefilled syringe 600 is the same as that of the prefilled syringe 200, and thus the description thereof will be omitted.

In the prefilled syringe 200 or 600 or the liquid medicine administration system 1 according to the embodiment of the present disclosure, the outer diameter D of the body section 202a of the barrel 202 is 14 mm to 33 mm. Further, the maximum circumferential length Lc of the antenna of the RFID tag 206 along the circumferential direction C of the body section 202a of the barrel 202 is 9 mm to 25 mm. Furthermore, the circumferential length of the outer circumferential surface of the body section 202a is 2.0 to 7.0 times the maximum circumferential length of the antenna of the RFID tag 206.

With these configurations, even if the RFID tag 206 of the prefilled syringe 200 and the reader of the syringe pump 100 are not aligned when the cylindrical prefilled syringe 200 or 600 is mounted on the supporting section 4 of the syringe pump 100, the RFID tag 206 can communicate with the reader 31. Further, since the positional alignment of the prefilled syringe 200 or 600 is unnecessary, it is possible to prevent the RFID tag 206 fixed to the outer circumferential surface of the prefilled syringe 200 or 600 from being damaged because of the sliding movement between the RFID tag 206 of the prefilled syringe 200 or 600 and the supporting section 4 of the syringe pump 100.

In addition, when the RFID tag is attached to the outer circumferential surface of the prefilled syringe, the attached RFID tag may be deformed and detached, because the outer circumferential surface of the prefilled syringe is curved. On the other hand, due to the configuration described above, detachment of the RFID tag 206 attached to the outer circumferential surface of the prefilled syringe 200 or 600 can be prevented.

In the prefilled syringe 200 or 600 according to the embodiment of the present disclosure, the maximum axial length La of the RFID tag 206 along the axial direction of the body section 202a of the barrel 202 is 8 mm to 25 mm. Due to the maximum axial length La of the RFID tag 206 being set to 8 mm or more, the RFID tag 206 can more reliably communicate with the reader of the syringe pump 100 when the cylindrical prefilled syringe 200 or 600 is mounted on the supporting section 4 of the syringe pump 100. Further, due to the maximum axial length La of the RFID tag 206 being set to 25 mm or less, it is possible to prevent the scale 301 from being difficult to see by the RFID tag 206, and to prevent the RFID tag 206 from deteriorating visibility of the liquid medicine 201 contained in the body section 202a.

In the prefilled syringe 200 or 600 according to the embodiment of the present disclosure, the barrel 202 has the flange 202c protruding from the proximal end section of the body section 202a, the body section 202a has, near the flange 202c, the clamp contact portion 209 where the clamp section 5 of the syringe pump 100 that drives the plunger 205 contacts, and the RFID tag 206 is located distal to the clamp contact portion 209 in the axial direction A of the body section 202a. This prevents the clamp section 5 of the syringe pump 100 from contacting the RFID tag 206, thereby being capable of preventing the RFID tag 206 from being damaged.

In the prefilled syringe 200 or 600 according to the embodiment of the present disclosure, the distance between the proximal end of the RFID tag 206 and the distal end of the flange 202c of the barrel 202 in the axial direction of the body section 202a is 15 mm to 40 mm. Due to the configuration in which the distance between the proximal end of the RFID tag 206 and the distal end of the flange 202c of the barrel 202 is set to 15 mm or more in the axial direction of the body section 202a, the contact between the clamp section 5 of the syringe pump 100 and the RFID tag 206 can be prevented to prevent damage of the RFID tag 206. Further, due to the configuration in which the distance between the proximal end of the RFID tag 206 and the distal end of the flange 202c of the barrel 202 is set to 40 mm or less in the axial direction of the body section 202a, it is possible to prevent the scale 301 from being difficult to see and to ensure visibility of the liquid medicine 201 contained in the body section 202a.

In the prefilled syringe 200 or 600 according to the embodiment of the present disclosure, the RFID tag 206 is located proximal to the distal end of the gasket 204 in the axial direction A of the body section 202a. This configuration can prevent the scale 301 from being difficult to see, and can ensure visibility of the liquid medicine 201 contained in the body section 202a.

The prefilled syringe 200 according to the embodiment of the present disclosure further includes the information label 300 attached to the body section 202a of the barrel 202. The RFID tag 206 can be attached to the inner surface or the outer surface of the information label 300. With these configurations, when the prefilled syringe 200 is manufactured, the information label 300 and the RFID tag 206 can be attached to the barrel 202 at one time as shown in Fig. 5, whereby the production efficiency of the prefilled syringe 200 can be improved.

The prefilled syringe 200 may be configured such that the information label 300 is attached to the body section 202a of the barrel 202 so as to cover the outer surface of the RFID tag 206. This can prevent the RFID tag 206 fixed to the outer circumferential surface of the body section 202a from being detached.

In the prefilled syringe 200 or 600 according to the embodiment of the present disclosure, the information label 300 has the scale 301 indicating an amount of the liquid medicine 201 in the body section 202a, the scale 301 being provided along the axial direction A of the body section 202a of the barrel 202, and at least a part of the RFID tag 206 is located at a position overlapping with at least a part of the scale 301 in the axial direction A of the body section 202a of the barrel 202 and displaced in the circumferential direction C of the body section 202a of the barrel 202. This makes it possible to provide the scale 301 and the RFID tag 206 on the prefilled syringe 200 or 600 without increasing the axial length of the prefilled syringe 200 or 600.

In the prefilled syringe 200 or 600 according to the embodiment of the present disclosure, a plurality of scales 301 is provided, and the RFID tag 206 is located between at least two scales 301. With this configuration, when the prefilled syringe 200 or 600 is mounted on the syringe pump 100, at least one scale 301 can be exposed and visually recognized.

In the prefilled syringe 200 or 600 according to the embodiment of the present disclosure, at least two scales 301 are symmetrical with respect to the central axis of the body section 202a of the barrel 202. With this configuration, at least one scale 301 can be exposed and visually recognized more reliably.

In the liquid medicine administration system 1 according to the embodiment of the present disclosure, the syringe pump 100 has the clamp section 5 that faces the supporting section 4 and clamps the body section 202a of the prefilled syringe 200 or 600 with the supporting section 4, and when the clamp section 5 clamps the prefilled syringe 200 or 600 between the supporting section 4 and the clamp section 5, the clamp contact portion 209 which is a region clamped by the clamp section 5 in the outer circumferential surface of the body section 202a is different from a region where the RFID tag 206 is attached. This prevents the clamp section 5 of the syringe pump 100 from contacting the RFID tag 206, thereby being capable of preventing the RFID tag 206 from being damaged.

In the liquid medicine administration system 1 according to the embodiment of the present disclosure, the barrel 202 has the flange 202c that protrudes from the proximal end section of the body section 202a, a distance between the proximal end of the RFID tag 206 and the distal end of the flange 202c of the barrel 202 is 15 mm to 40 mm in the axial direction of the body section 202a, and the clamp section 5 comes in contact with a vicinity of the flange 202c of the body section 202a, and clamps the prefilled syringe 200 or 600 with the supporting section 4. Due to the configuration in which the distance between the proximal end of the RFID tag 206 and the distal end of the flange 202c of the barrel 202 is set to 15 mm or more in the axial direction of the body section 202a, and the clamp section 5 of the syringe pump 100 comes in contact with the clamp contact portion 209 which is the vicinity of the flange 202c of the body section 202a, the contact between the clamp section 5 and the RFID tag 206 can be prevented to prevent damage of the RFID tag 206. Further, due to the configuration in which the distance between the proximal end of the RFID tag 206 and the distal end of the flange 202c of the barrel 202 is set to 40 mm or less in the axial direction of the body section 202a, it is possible to prevent the scale 301 from being difficult to see and to ensure visibility of the liquid medicine 201 contained in the body section 202a.

In the liquid medicine administration system 1 according to the embodiment of the present disclosure, the main body 3 of the syringe pump 100 has the first region 3a1 facing the clamp section 5 and the second region 3a2 that is adjacent to the first region 3a1 and is located on a side opposite to the syringe plunger driving section 2 in the axial direction A of the body section 202a, the reader 31 includes the reader antenna 31a that communicates with the antenna 206a of the RFID tag 206 and that is structured from a reader antenna wire, and at least a part of the reader antenna 31a is located in the second region 3a2. That is, at least a part of the reader antenna 31a is arranged in the second region 3a2, which is not the first region 3a1 facing the clamp section 5. With this configuration, the reader antenna 31a can reliably read the data set of the RFID tag 206. Note that the prefilled syringe 200 or 600 is mounted to the syringe pump 100 such that at least a part of the RFID tag 206 faces the second region 3a2.

In the liquid medicine administration system 1 according to the embodiment of the present disclosure, the centerline lca of the reader antenna 31a perpendicular to the axial direction of the body section 202a of the barrel 202 is located in the second region 3a2 in a front view of the supporting section 4, and the centerline lca of the reader antenna 31a overlaps with the antenna of the RFID tag 206 in the front view of the supporting section 4. Here, it is preferable that the position of the centerline lca of the reader antenna 31a substantially coincides with the position where the magnetic flux density of the magnetic flux generated by the reader antenna 31a is the highest. Therefore, due to the configuration in which the centerline lca of the reader antenna 31a overlaps with the antenna of the RFID tag 206, the reader 31 can more reliably read the data set of the RFID tag 206.

The syringe pump 100 according to the embodiment of the present disclosure includes the clamp section 5 that faces the supporting section 4 and clamps the body section 202a of the prefilled syringe 200 with the supporting section 4, the main body 3 of the syringe pump 100 has the first region 3a1 facing the clamp section 5 of the body section 202a and the second region 3a2 that is adjacent to the first region 3a1 and is located on a side opposite to the syringe plunger driving section 2 in the axial direction A of the body section 202a, the reader 31 includes the reader antenna 31a that communicates with the antenna of the RFID tag 206 and that is structured from a reader antenna wire, and at least a part of the reader antenna 31a is located in the second region 3a2. With this configuration, at least a part of the reader antenna 31a is arranged in the second region 3a2, which is not the first region 3a1 facing the clamp section 5, so that the reader antenna 31a can read the data set of the RFID tag 206. Further, when the prefilled syringe 200 is clamped by the supporting section 4 and the clamp section 5, the RFID tag 206 can be prevented from being damaged due to contact with the clamp section 5. Note that the prefilled syringe 200 or 600 is mounted to the syringe pump 100 such that at least a part of the RFID tag 206 faces the second region 3a2.

Further, in the syringe pump 100 according to the embodiment of the present disclosure, the centerline lca of the reader antenna 31a, which is perpendicular to the body section 202a of the barrel 202, is located in the second region 3a2 in a front view of the supporting section 4. This allows the reader 31 to reliably read the data of the RFID tag 206 of the syringe pump 100.

### [Examples]

Next, prefilled syringes according to the embodiment of the present disclosure were manufactured and their performance was evaluated. This will be described below.

In this example, six prefilled syringes 1 to 6 having different outer diameters of body sections of barrels were prepared. The outer diameters of the body sections of the barrels of the prefilled syringes 1 to 6 were 11 mm, 14 mm, 17 mm, 22 mm, 33 mm, and 35.5 mm, respectively.

### <Verification of attachment of RFID tag>

RFID tags 1 to 6 were attached to the outer circumferential surfaces of the body sections of the barrels of the prefilled syringes 1 to 6, and then, whether the RFID tags 1 to 6 were detached or not was checked. In each of the RFID tags 1 to 5, the shape of the tag body including an antenna, a memory, and a control unit and the outer perimeter of an antenna wire are rectangular. Further, the shape of a tag body of the RFID tag 6 is a circle with an outer diameter of 28 mm, and the outer perimeter of an antenna wire is a circle with an outer diameter of 25 mm. Table 1 shows the dimensions of the tag bodies of the RFID tags 1 to 6 and the dimensions of the antennas. Table 1 also shows values (indicated as "syringe-tag circumferential length ratios" in Table 1) each obtained by dividing the circumferential length of the outer circumferential surface of the body section of the barrel of prefilled syringe by the maximum circumferential length of the antenna of the RFID tag. In this example, the RFID tags 1 to 6 were attached to the outer circumferential surfaces of the body sections of the prefilled syringes 1 to 6, respectively, and then, it was checked whether the RFID tags 1 to 6 were detached. "○'' in Table 1 shows the case where the RFID tag was not detached. "X" indicates that the RFID tag could not be attached due to its large size, or that the RFID tag was attached and then detached, in other words, the RFID tag could not be properly attached to the prefilled syringe.

### <Verification regarding reading of RFID tag>

The RFID tags 1 to 6 were attached to the outer circumferential surfaces of the body sections of the barrels of the prefilled syringes 1 to 6, and each of the prefilled syringes 1 to 6 was mounted on the syringe pump 100 shown in Fig. 1. Here, the prefilled syringes 1 to 6 were mounted such that the attached RFID tags 1 to 6 were at positions farthest from the reader 31 of the syringe pump 100, that is, the RFID tag 206 faced the front side in Fig. 1. In such a state, it was checked multiple times whether the reader 31 of the syringe pump 100 could read the data of the RFID tags 1 to 6. "Excellent" in Table 1 indicates that the RFID tag data could be read in all cases. "Good" indicates that the RFID tag data could not be read in some cases. Furthermore, "-" indicates that the test was not performed because the RFID tag could not be properly attached to the outer circumferential surface of the body section of the barrel of the prefilled syringe.

**[Table 1]**

| | | RFID tag 1 | RFID tag 2 | RFID tag 3 | RFID tag 4 | RFID tag 5 | RFID tag 6 |
|---|---|---|---|---|---|---|---|
| axial length of RFID tag body [mm] | | 19 | 18 | 28 | 18 | 35 | 28 (circle) |
| circumferential length of RFID tag body [mm] | | 13 | 18 | 28 | 50 | 65 | |
| axial length of antenna [mm] | | 12 | 15 | 25 | 8 | 30 | 25 (circle) |
| circumferential length of antenna [mm] | | 9 | 15 | 25 | 37 | 60 | |
| prefilled syringe 1 (capacity: 3 ml, outer diameter: 11 mm) | syringe-tag circumferential length ratio | 3.8 | 2.3 | 1.4 | 0.93 | 0.58 | 1.4 |
| | attachment of RFID tag | Excellent | Excellent | No good | No good | No good | No good |
| | reading of RFID tag | Good | Good | - | - | - | - |
| prefilled syringe 2 (capacity: 5 ml, outer diameter: 14 mm) | syringe-tag circumferential length ratio | 4.9 | 2.9 | 1.8 | 1.2 | 0.73 | 1.8 |
| | attachment of RFID tag | Excellent | Excellent | No good | No good | No good | No good |
| | reading of RFID tag | Excellent | Excellent | - | - | - | - |
| prefilled syringe 3 (capacity: 10 ml, outer diameter: 17 mm) | syringe-tag circumferential length ratio | 5.9 | 3.6 | 2.1 | 1.4 | 0.89 | 2.1 |
| | attachment of RFID tag | Excellent | Excellent | Excellent | No good | No good | Excellent |
| | reading of RFID tag | Excellent | Excellent | Excellent | - | - | Good |
| prefilled syringe 4 (capacity: 20 ml, outer diameter: 22 mm) | syringe-tag circumferential length ratio | 7.7 | 4.6 | 2.8 | 1.9 | 1.2 | 2.8 |
| | attachment of RFID tag | Excellent | Excellent | Excellent | No good | No good | Excellent |
| | reading of RFID tag | Excellent | Excellent | Excellent | - | - | Good |
| prefilled syringe 5 (capacity: 50 ml, outer diameter: 33 mm) | syringe-tag circumferential length ratio | 11.5 | 6.9 | 4.1 | 2.8 | 1.7 | 4.1 |
| | attachment of RFID tag | Excellent | Excellent | Excellent | Excellent | No good | Excellent |
| | reading of RFID tag | Excellent | Excellent | Excellent | Good | - | Good |
| prefilled syringe 6 (capacity: 100 ml, outer diameter: 35.5 mm) | syringe-tag circumferential length ratio | 12.4 | 7.4 | 4.5 | 3.0 | 1.9 | 4.5 |
| | attachment of RFID tag | Excellent | Excellent | Excellent | Excellent | No good | Excellent |
| | reading of RFID tag | Good | Good | Good | Good | - | Good |

As is clear from Table 1, in prefilled syringes each configured such that the antenna is structured from an antenna wire wound in a rectangle, the outer diameter of the body section of the barrel is 14 mm to 33 mm, the maximum circumferential length Lc of the antenna of the RFID tag along the circumferential direction of the body section a of the barrel is 9 mm to 25 mm, and the circumferential length of the outer circumferential surface of the body section is set to be 2.0 to 7.0 times the maximum circumferential length of the antenna of the RFID tag 206, the syringe pump more easily acquires data, and the attached RFID tag is less likely to be detached, as compared to the other prefilled syringes.

### Reference Signs List

1 Liquid medicine administration system
200, 600 Prefilled syringe
201 Liquid medicine
202 Barrel
202a Body section
202b Nozzle section
202b1 Distal end opening
202c Flange
203 Cap
204 Gasket
205 Plunger
205a Plunger body
205b Flange
206 RFID tag
206a Antenna
206b Memory
206c Control unit
207 Tube
208 Indwelling needle
209 Clamp contact portion
100 Syringe pump
13 Control unit
2 Syringe plunger driving section
2a Pressing part
2b Flange fixing part
3 Main body
31 Reader
31a Reader antenna
31b Memory
31c Control unit
32 Display unit
33 Operation panel
33A Power switch
33B Fast delivery switch
33C Start switch
33D Stop switch
33E Display changeover switch
33F Mute switch
33G Confirmation switch
33H Operation indicator
3a1 First region
3a2 Second region
4 Supporting section
5 Clamp section
6 Base
7 Flange receiving groove
300, 400 Information label
301 Scale
302 Information area
A Axial direction of body section
B Radial direction of body section
C Circumferential direction of body section
D Outer diameter of body section
La Axial length of RFID tag
Lc Circumferential length of RFID tag
O2 Central axis
P Patient
lca Centerline

## Claims

1. A syringe pump (100) to which a prefilled syringe (200; 600) is mounted,
the prefilled syringe (200; 600) including
a liquid medicine (201),
a barrel (202) including a body section (202a) that is cylindrical and that contains the liquid medicine (201), and a nozzle section (202b) that is provided on a distal end side of the body section (202a) and that discharges the liquid medicine (201),
a cap (203) that seals a distal end opening (202b1) provided on a distal end section of the nozzle section (202b),
a gasket (204) that slides on an inner circumferential surface of the body section (202a),
a plunger (205) mountable to the gasket (204), and
an RFID tag (206) that has a fixed location with respect to an outer circumferential surface of the body section (202a) and has an antenna (206a) for communication and a memory (206b), the antenna (206a) being structured from an antenna wire that is wound in a rectangle,
wherein an outer diameter of the body section (202a) of the barrel (202) is 14 mm to 33 mm,
a maximum circumferential length of the antenna (206a) of the RFID tag along a circumferential direction of the body section (202a) of the barrel (202) is 9 mm to 25 mm, and
a circumferential length of the outer circumferential surface of the body section (202a) of the barrel is 2.0 to 7.0 times the maximum circumferential length of the antenna (206a) of the RFID tag,
the syringe pump (100) comprising:
a syringe plunger driving section (2) that drives the plunger (205) of the prefilled syringe (200; 600) in a distal direction toward the distal end of the body section (202a);
a main body (3) provided with a reader (31) and a control unit (31c) that controls the syringe plunger driving section (2), the reader (31) reading a data set stored in the memory (206b) of the RFID tag (206) of the prefilled syringe (200; 600);
a supporting section (4) that supports the outer circumferential surface of the body section (202a) of the prefilled syringe (200; 600) in a direction perpendicular to an axis of the body section (202a); and
a clamp section (5) that faces the supporting section (4) and clamps the body section (202a) of the prefilled syringe (200; 600) with the supporting section (4),
wherein the main body (3) of the syringe pump (100) has a first region (3a1) facing the clamp section (5) and a second region (3a2) that is adjacent to the first region (3a1) and is located on a side opposite to the syringe plunger driving section (2) in an axial direction of the body section (202a),
the reader (31) includes a reader antenna (31a) that communicates with the antenna (206a) of the RFID tag (206) and that is structured from a reader antenna wire, and
at least a part of the reader antenna (31a) is located in the second region (3a2),
wherein a centerline (lca) of the reader antenna (31a) perpendicular to the axial direction of the body section (202a) of the barrel (202) is located in the second region (3a2) in a front view of the supporting section (4), and
at least a part of the reader antenna (31a) overlaps with the antenna (206a) of the RFID tag (206) in the front view of the supporting section (4).

2. The syringe pump (100) according to claim 1, wherein the centerline (lca) of the reader antenna (31a) overlaps with the antenna (206a) of the RFID tag (206) in the front view of the supporting section (4).

## Patentansprüche

1. Spritzenpumpe (100), an der eine vorgefüllte Spritze (200; 600) montiert ist, wobei
die vorgefüllte Spritze (200; 600) hat
ein flüssiges Arzneimittel (201),
einen Zylinder (202) mit einem zylindrischen Körperabschnitt (202a), der das flüssige Arzneimittel (201) enthält, und einem Düsenabschnitt (202b), der an einer distalen Endseite des Körperabschnitts (202a) bereitgestellt ist und das flüssige Arzneimittel (201) abgibt,
eine Abdeckung (203), die eine distale Endöffnung (202b1) abdichtet, die an einem distalen Endabschnitt des Düsenabschnitts (202b) bereitgestellt ist,
eine Dichtung (204), die auf einer Innenumfangsfläche des Körperabschnitts (202a) gleitet,
einen Kolben (205), der an der Dichtung (204) montierbar ist, und
ein RFID-Etikett (206), das eine feste Position mit Bezug auf eine Außenumfangsfläche des Körperabschnitts (202a) aufweist und eine Antenne (206a) zur Kommunikation und einen Speicher (206b) aufweist, wobei
die Antenne (206a) aus einem Antennendraht strukturiert ist, der in einem Rechteck gewickelt ist, wobei
ein Außendurchmesser des Körperabschnitts (202a) des Zylinders (202) 14 mm bis 33 mm beträgt,
eine maximale Umfangslänge der Antenne (206a) des RFID-Tags entlang einer Umfangsrichtung des Körperabschnitts (202a) des Zylinders (202) 9 mm bis 25 mm beträgt, und
eine Umfangslänge der Außenumfangsfläche des Körperabschnitts (202a) des Zylinders das 2,0- bis 7,0-fache der maximalen Umfangslänge der Antenne (206a) des RFID-Tags beträgt, wobei
die Spritzenpumpe (100) aufweist:
einen Spritzenkolbenantriebsabschnitt (2), der den Kolben (205) der vorgefüllten Spritze (200; 600) in einer distalen Richtung zum distalen Ende des Körperabschnitts (202a) antreibt;
einen Hauptkörper (3), der mit einem Lesegerät (31) und einer Steuereinheit (31c) versehen ist, die den Spritzenkolbenantriebsabschnitt (2) steuert, wobei das Lesegerät (31) einen Datensatz liest, der in dem Speicher (206b) des RFID-Etiketts (206) der vorgefüllten Spritze (200; 600) gespeichert ist;
einen Halteabschnitt (4), der die außenliegende Umfangsfläche des Körperabschnitts (202a) der vorgefüllten Spritze (200; 600) in einer Richtung senkrecht zu einer Achse des Körperabschnitts (202a) hält; und
einen Klemmabschnitt (5), der dem Halteabschnitt (4) gegenüberliegt und den Körperabschnitt (202a) der vorgefüllten Spritze (200; 600) mit dem Halteabschnitt (4) klemmt,
wobei der Hauptkörper (3) der Spritzenpumpe (100) einen ersten Bereich (3a1), der dem Klemmabschnitt (5) zugewandt ist, und einen zweiten Bereich (3a2) aufweist, der an den ersten Bereich (3a1) angrenzt und auf einer Seite gegenüber dem Spritzenkolben-Antriebsabschnitt (2) in einer axialen Richtung des Körperabschnitts (202a) angeordnet ist,
der Leser (31) eine Leserantenne (31a) hat, die mit der Antenne (206a) des RFID-Etiketts (206) kommuniziert und die aus einem Leserantennenleiter strukturiert ist, und
zumindest ein Teil der Leserantenne (31a) in dem zweiten Bereich (3a2) angeordnet ist,
eine Mittellinie (lca) der Leserantenne (31a) senkrecht zu der axialen Richtung des Körperabschnitts (202a) des Zylinders (202) in einer Vorderansicht des Halteabschnitts (4) in dem zweiten Bereich (3a2) angeordnet ist, und
zumindest ein Teil der Leseantenne (31a) in der Vorderansicht des Halteabschnitts (4) mit der Antenne (206a) des RFID-Tags (206) überlappt.

2. Spritzenpumpe (100) nach Anspruch 1, wobei die Mittellinie (lca) der Leseantenne (31a) in der Vorderansicht des Halteabschnitts (4) mit der Antenne (206a) des RFID-Tags (206) überlappt.

## Revendications

1. Pompe à seringue (100) sur laquelle est montée une seringue préremplie (200 ; 600),
la seringue préremplie (200 ; 600) comprenant
un médicament liquide (201),
un tube (202) comprenant une section de corps (202a) qui est cylindrique et qui contient le médicament liquide (201), et une section de buse (202b) qui est prévue sur un côté d'extrémité distale de la section de corps (202a) et qui décharge le médicament liquide (201),
un capuchon (203) qui scelle une ouverture d'extrémité distale (202b1) prévue sur une section d'extrémité distale de la section de buse (202b),
un joint (204) qui glisse sur une surface circonférentielle intérieure de la section de corps (202a),
un piston (205) pouvant être monté sur le joint (204), et
une étiquette RFID (206) qui a un emplacement fixe par rapport à une surface circonférentielle extérieure de la section de corps (202a) et qui a une antenne (206a) pour la communication et une mémoire (206b), l'antenne (206a) étant structurée d'un fil d'antenne enroulé en rectangle,
dans laquelle un diamètre extérieur de la section de corps (202a) du tube (202) est compris entre 14 mm et 33 mm,
une longueur circonférentielle maximale de l'antenne (206a) de l'étiquette RFID le long d'une direction circonférentielle de la section de corps (202a) du tube (202) est comprise entre 9 mm et 25 mm, et
une longueur circonférentielle de la surface circonférentielle extérieure de la section de corps (202a) du tube est comprise entre 2,0 et 7,0 fois la longueur circonférentielle maximale de l'antenne (206a) de l'étiquette RFID,
la pompe à seringue (100) comprenant :
une section d'entraînement du piston de la seringue (2) qui entraîne le piston (205) de la seringue préremplie (200 ; 600) dans une direction distale vers l'extrémité distale de la section de corps (202a) ;
un corps principal (3) équipé d'un lecteur (31) et d'une unité de commande (31c) qui commande la section d'entraînement du piston de la seringue (2), le lecteur (31) lisant un ensemble de données stockées dans la mémoire (206b) de l'étiquette RFID (206) de la seringue préremplie (200 ; 600) ;
une section de support (4) qui supporte la surface circonférentielle extérieure de la section de corps (202a) de la seringue préremplie (200 ; 600) dans une direction perpendiculaire à un axe de la section de corps (202a) ; et
une section de serrage (5) qui fait face à la section de support (4) et qui serre la section de corps (202a) de la seringue préremplie (200 ; 600) avec la section de support (4),
dans laquelle le corps principal (3) de la pompe à seringue (100) a une première zone (3a1) faisant face à la section de serrage (5) et une seconde zone (3a2) adjacente à la première zone (3a1) et située sur un côté opposé à la section d'entraînement du piston de la seringue (2) dans une direction axiale de la section de corps (202a),
le lecteur (31) comprend une antenne de lecteur (31a) qui communique avec l'antenne (206a) de l'étiquette RFID (206) et qui est structurée d'un fil d'antenne de lecteur, et
au moins une partie de l'antenne de lecteur (31a) est située dans la seconde zone (3a2),
dans laquelle une ligne central (lca) de l'antenne de lecteur (31a) perpendiculaire à la direction axiale de la section de corps (202a) du tube (202) est situé dans la seconde zone (3a2) dans une vue de face de la section de support (4), et
au moins une partie de l'antenne de lecteur (31a) chevauche l'antenne (206a) de l'étiquette RFID (206) dans la vue de face de la section de support (4).

2. Pompe à seringue (100) selon la revendication 1, dans laquelle la ligne centrale (lca) de l'antenne de lecteur (31a) chevauche l'antenne (206a) de l'étiquette RFID (206) dans la vue de face de la section de support (4).
